# EUROPEAN PATENT APPLICATION

(11) **EP 4 406 634 A1**
(43) Date of publication of application: **31.07.2024**
(21) Application number: 23153884.4
(22) Date of filing: 30.01.2023
(51) Int. Cl.: B01D 15/34, B01D 15/12, G01N 30/02, C07K 1/16

(54) **A PROCESS FOR SIZE EXCLUSION CHROMATOGRAPHY OF A NOT FULLY SOLUBLE POLYMERIC SUBSTANCE, AND A REFERENCE SUBSTANCE FOR SIZE EXCLUSION CHROMATOGRAPHY OF A POLYMERIC SUBSTANCE**

(71) Applicant: BASF SE, 67056 Ludwigshafen am Rhein (DE)
(72) Inventor: Beskers, Timo Florian, 67056 Ludwigshafen am Rhein (DE); Czamy, Bianca, 67056 Ludwigshafen am Rhein (DE); Staal, Bastiaan Bram Pieter, 67056 Ludwigshafen am Rhein (DE)
(74) Representative: Schuck, Alexander

(57) **Abstract**

A process for size exclusion chromatography of a polymeric substance 1 comprising the following steps a) Mixing the polymeric substance 1 with a solvent, whereby a mixture M1 is obtained; b) Ultrasonic treatment of at least a part of the mixture M1 obtained in step a), which is named mixture M1A, whereby a mixture M2A comprising a polymeric substance 2 is obtained; and c) Subjecting the mixture M2A obtained in step b) to size exclusion chromatography, at a given temperature and at a given concentration, wherein the polymeric substance 1 is not fully soluble in the solvent at the temperature and concentration in step c); a reference substance, of a polymeric substance 1, obtainable by a) mixing the polymeric substance 1 with a solvent, whereby a mixture M1 is obtained; and b) ultrasonic treatment of the mixture M1 obtained in step a), whereby the reference standard for the polymeric substance 1 is obtained; a size exclusion chromatography column comprising the reference standard and the use of the polymeric substance 2 obtained in step b) of the process according to the present invention as a reference substance for polymeric substance 1.

## Description

The present invention relates to a process for size exclusion chromatography of a polymeric substance 1 comprising the following steps a) Mixing the polymeric substance 1 with a solvent, whereby a mixture M1 is obtained; b) Ultrasonic treatment of at least a part of the mixture M1 obtained in step a), which is named mixture M1A, whereby a mixture M2A comprising a polymeric substance 2 is obtained; and c) Subjecting the mixture M2A obtained in step b) to size exclusion chromatography, at a given temperature and at a given concentration, wherein the polymeric substance 1 is not fully soluble in the solvent at the temperature and concentration in step c); a reference substance, of a polymeric substance 1, obtainable by a) mixing the polymeric substance 1 with a solvent, whereby a mixture M1 is obtained; and b) ultrasonic treatment of the mixture M1 obtained in step a), whereby the reference standard for the polymeric substance 1 is obtained; a size exclusion chromatography column comprising the reference standard and the use of the polymeric substance 2 obtained in step b) of the process according to the present invention as a reference substance for polymeric substance 1.

Size exclusion chromatography (SEC) also called gel permeation chromatography (GPC), gel filtration chromatography (GFC) or molecular sieve chromatography is a liquid chromatography (LC) technique to separate polymers based on their size in solution. From an SEC chromatogram, for example the proportion of the molecular weight distribution of the macromolecules forming the polymer can be determined via the peak area. For regulatory purposes, the lower molecular weight fractions, mostly smaller than 1000 g/mol and often less than 500 g/mol, are important parameters, for example for assessing the toxicology of the polymers. Exact methods for determination of the low molecular weight content of a polymer, the number-average molecular weight and the molecular weight distribution of polymers are described in OECD Guidelines 118 (OECD (1996), Test No. 118: Determination of the Number-Average Molecular Weight and the Molecular Weight Distribution of Polymers using Gel Permeation Chromatography, OECD Guidelines for the Testing of Chemicals, Section 1, OECD Publishing, Paris, https://doi.org/10.1787/9789264069848-en) and 119 (OECD (1996), Test No. 119: Determination of the Low Molecular Weight Content of a Polymer Using Gel Permeation Chromatography, OECD Guidelines for the Testing of Chemicals, Section 1, OECD Publishing, Paris, https://doi.org/10.1787/9789264069862-en). These guidelines are based on DIN and ISO norms of similar content (DIN EN ISO 13885-1 to 3 and ISO 16014-1 to 5).

The OECD Guidelines for the Testing of Chemicals is a collection of about 150 of the most relevant internationally agreed testing methods used by government, industry and independent laboratories to identify and characterize potential hazards of chemicals. They are a set of tools for professionals, used primarily in regulatory safety testing and subsequent chemical and chemical product notification, chemical registration and in chemical evaluation. They can also be used for the selection and ranking of candidate chemicals during the development of new chemicals and products and in toxicology research. This group of tests covers physical-chemical properties.

It is known in the art that depending on the solubility of the polymer, different solvents and column materials can be used. Many polymers can be analyzed in this way.

In SEC, the separation takes place in a so-called separation column which is packed with porous particles with a diameter of typically 1 µm to 20 µm. The volume between these particles therefore determines, in addition to the largest pores present, the upper limit of the SEC separation. Macromolecules or species of the polymers which are insoluble in the solvent used in the SEC, such as those found for example in very high molecular weight or cross-linked samples, cannot pass through the SEC separation column and must be filtered out to protect the column from clogging. In such samples, only the soluble fraction can be analyzed, thus underestimating the molecular weight distribution and its mean values (Mₙ (number average molecular weight), M_{w} (weight average molecular weight), M_{z} (z average molecular weight), wherein n refers to the number, w refers to the weight and z refers to the German word "Zentrifuge", which is an average which can be determined by ultracentrifugation) and overestimating the low molecular weight fractions. It is difficult to determine the proportion of the analyzed sample from the total sample. Weighing the filter has too much inaccuracy. It is better to calibrate the detector response for concentration to determine the amount of the analyzed sample (usually, SEC is only calibrated for molecular weight), as also recommended in the Annex of OECD testing guideline 119. However, this requires a completely soluble, SEC-compatible reference substance of identical chemical composition to the sample. This is usually not available nor synthesizable. Since the detector response of the most commonly used RI detector (differential refractive index detector) depends on the dn/dc value of the sample, one can - where available - fall back on literature values, which are often inaccurate or not reliable. The dn/dc value (refractive index increment) refers to the rate of change of the refractive index with the concentration of a solution for a sample at a given temperature, a given wavelength, and a given solvent. Measurement of the response factor with another device (e.g. a refractometer in case of RI detection) can be performed, but the values measured in batch are for example influenced by side components and the solvent. As SEC separates the polymer from side components, those values should not be used.

In summary, the analysis of polymer samples which are not completely soluble at a given temperature in a given solvent by SEC is difficult and the determination of the low molecular weight fractions according to OECD 118 and 119, as required for example for the registration of such products in various countries, is often not possible. A suitable alternative, an equivalent method for determination does not exist.

One object of the present invention is therefore the provision of a method for obtaining reliable data about the low molecular weight fractions, the molecular weight distribution and the molecular weight averages (Mₙ, M_{w}, M_{z}) of polymers which are not completely soluble at a given temperature in a given solvent, especially cross-linked or high-molecular weight polymers, by SEC.

The primary information obtained by standard SEC detectors (RI, but also UV, ELSD (Evaporative Light Scattering Detector)) is not the molecular weight but the apparent concentration at a certain elution volume. A combination of a calibration curve and concentration profile from a concentration detector is required to calculate all molecular weight averages and molecular weight distributions. SEC is therefore regarded as a relative technique. The most popular method for calibration is the method that utilizes narrow distribution standard polymers. However, the calibration is only accurate for polymers of the same structural and chemical nature as the polymers to be analyzed and narrow distribution standards are not available for all polymer types. To quantify the amount of sample that reaches the detector an additional calibration of the detector constant is needed. Again, suitable reference standards are often not available.

It is a further object of the present invention to provide a reference substance (calibration standard) which is readily available for any polymer to be analyzed by SEC and can be used to determine the detector constant.

The objects mentioned above are solved by a process for size exclusion chromatography (SEC) of a polymeric substance 1, comprising the following steps:
a) mixing the polymeric substance 1 with a solvent, whereby a mixture M1 is obtained;
b) ultrasonic treatment of at least a part of the mixture M1 obtained in step a), which is named mixture M1A, whereby a mixture M2A comprising a polymeric substance 2 is obtained; and
c) subjecting the mixture M2A obtained in step b) to size exclusion chromatography at a given temperature and at a given concentration,
wherein the polymeric substance 1 is not fully soluble in the solvent at the temperature and concentration in step c).

The objects are further solved by a reference substance (calibration standard), preferably for size exclusion chromatography, of a polymeric substance 1, obtainable by
a) mixing the polymeric substance 1 with a solvent, whereby a mixture M1 is obtained; and
b) ultrasonic treatment of the mixture M1 obtained in step a), whereby the reference standard for the polymeric substance 1 is obtained.

The object is further solved by an SEC column comprising the inventive reference standard and by the use of a polymeric substance 2 obtained in step b) of the process according to the present invention as a reference standard for polymeric substance 1, preferably in size exclusion chromatography.

US2008162055A1 relates to a process for determining a molecular weight distribution in a polymer, comprising measuring the complex shear modulus as a function of the frequency, fitting an analytical function to the measured complex shear modulus or to the mastercurve determined from the measured complex shear modulus, and calculating the molecular weight distribution therefrom. It is mentioned in US2008162055A1 that the GPC (SEC) process for determining a molecular weight distribution has the disadvantage of only being able to analyze those polymers which are soluble. However, the process disclosed in US2008162055A1 is elaborated and time consuming and not recognized by authorities for the registration of substances.

There is no related art solving the objects mentioned above by SEC. However, the combination of ultrasonic irradiation and SEC is mentioned in the prior art but in a different context than in the present invention.

JP2009103635A relates to a method for analyzing a high molecular weight compound. The problem addressed in JP2009103635A is that MALDI-MS is difficult for polymers having a molecular weight of more than about 5000 g/mol due to a limitation in the analysis principle of MALDI-MS. The method according to JP2009103635A comprises therefore a first step of reducing the molecular weight of the high-molecular compound by ultrasonic irradiation and a further step of performing the mass spectrometry. Optionally, a fractionation step of fractionating the decomposition product of ultrasonic irradiation by size exclusion chromatography is performed after the ultrasonic irradiation. However, the size exclusion chromatography according to JP2009103635A is carried out by employing the solution (completely dissolved) of the high molecular weight compound, which has been subjected to ultrasonic irradiation.

Striegel, Journal of Liquid Chromatography & Related Technologies, 31:3105-3114, 2008 relates to observations regarding on-column, flow-induced degradation during SEC analysis. The problem discussed in Striegel is the degradation of highly extended polymers in an SEC column, which results in a molecular weight distribution broader than originally expected. In a comparative experiment, an ultrasonic degradation of a polystyrene solution is compared with an on-column degradation, whereby the analysis of said solution (fully soluble polystyrene) is carried out by SEC.

Kulicke et al., Macromol. Chem. Phys. 2000, 201, 1976-1984 sets the task to prove whether a series of molecular weights which were produced by ultrasonic degradation is suitable for establishing structure-property relationships. The set of samples was characterized by SEC. The samples characterized by SEC are completely soluble in the eluent (solvent) used in SEC.

Pethrick et al., Polymer Degradation and Stability 68 (2000) 445-449 concerns the ultrasonic degradation of polystyrene solutions. Data are reported on the degradation of narrow molecular weight distribution polystyrenes dissolved in a good solvent toluene, in an attempt to gain a greater understanding of the mechanism of degradation by ultrasound. The change in molecular weight on exposure to the ultrasound was monitored by use of gel permeation chromatography and solution viscosity measurements.

In all related art documents mentioned above, a solution of a completely (fully) dissolved polymer is characterized by SEC.

However, it is an object of the present invention to provide an SEC method for polymers, which are not fully soluble in the solvent used in the SEC.

It has been found by the inventors of the present invention that by the process of the present invention, a more exact determination of the low molecular weight fraction of a polymer sample according to OECD 118 and 119 as required for the registration of polymers which are not fully soluble in the solvents used in the SEC in various countries can be provided.

By the ultrasonic treatment of the polymer which is not completely soluble in the solvent used for the SEC, a more soluble or even a completely soluble sample having an identical chemical composition and thus identical detector response - dn/dc in the case of the most commonly used RI detector - which can be more exactly or even completely analyzed by SEC can be provided.

The polymer samples aliquot M1 obtained in the mixing step a) can for example be split into two parts. One part, M1B, is filtered and analyzed directly by SEC. The second part (M1A, reference substance) is treated with ultrasound before it is also analyzed by SEC identically. From the ratio of the two chromatograms, the low molecular weight proportions are calculated exactly and a better (i.e. higher) lower limit (estimation) can be determined for the molecular weight mean values.

From the SEC analysis of the ultrasonically treated reference sample (M1A) alone, a lower limit for the molecular weight mean values and an upper limit for the low molecular weight fractions is also directly accessible, i.e. without comparison with the measurement of the soluble fraction of the original polymer sample. In many cases, this simplified analysis can already meet the regulatory requirements (especially of OECD 118 and 119), if the required limits are met.

The process of the present invention therefore allows the accurate determination of the low molecular weight fractions by SEC of polymers which are not fully soluble in the solvent used for the SEC in compliance with the regulatory requirements for example of OECD 118 and 119. Said SEC analysis of such polymers not fully soluble in the SEC solvent was formerly impossible.

Size exclusion chromatography (SEC) generally separates macromolecules based on their size in a column generally packed with different sized particles with different sized pores. It is generally not restricted to any polymers and/or solvents.

In the process for SEC of the present invention, a polymeric substance 1, which is not fully soluble in the solvent used in the size exclusion chromatography at a given temperature and a given concentration set for the SEC (step c)), is carried out.

The type of polymeric substance 1 itself is generally not restricted to a certain polymer class.

A polymer according to the present invention is a substance composed of macromolecules (see the IUPAC Gold Book definition). Generally, macromolecules are very large molecules with a molecular weight ranging from a few thousand as high as millions of Da (Dalton). The IUPAC Gold Book definition of a macromolecule is: "A molecule of high relative molecular mass, the structure of which essentially comprises the multiple repetition of units derived, actually or conceptionally, from molecules of low relative molecular mass". Generally, the average number of repetition units in the polymeric substance 1 according to the present invention is at least 100.

The terms "polymer" and "polymeric substance" include according to the present invention any kind of homopolymers (polymers made from identical monomer units) and copolymers (polymers made from more than one kind of monomer), which may be further modified, e.g. by grafting. The present invention can further be used for mixtures of polymers and mixtures of polymers with other substances.

The polymeric substance 1 is generally a cross-linked or high molecular weight polymer.

Preferably, at least 2 wt.%, preferably at least 5 wt.%, more preferably 5 wt.% to 95 wt.% of the polymeric substance 1 have a molecular weight of at least 5 000 000 Da, determined according to OECD118.

In a further preferred embodiment, the polymeric substance 1 has a degree of cross-linking of at least 0.001%, preferably of at least 0.005%, more preferably of 0.01 to 30%. The degree of crosslinking of the polymeric substance 1 can be determined by means of NMR relaxation measurements as described in WO2016150999A1. Specific measuring conditions are given in the example part of WO2016150999A1.

It is further possible that at least 2 wt.%, preferably at least 5 wt.%, more preferably 5 wt.% to 95 wt.% of the polymeric substance 1 have a molecular weight of at least 5 000 000 Da and that the polymeric substance 1 has a degree of cross-linking of at least 0.001 %, preferably of at least 0.005%, more preferably of 0.01 to 30%.

A suitable polymeric substance 1 is generally a natural or a synthetic polymer.

Natural polymers are for example polymers like proteins, starch, cellulose, polysaccharides, asphaltenes or DNA.

Synthetic polymers are for example thermoplastics, elastomers, thermosets or synthetic fibers.

Typical synthetic polymers are polyethylene, e.g. low density polyethylene (LDPE) and high density polyethylene (HDPE), polypropylene (PP), polyvinylchloride (PVC), polystyrene (PS), polyamide (PA), e.g. polyamide 6 and polyamide 6,6, Teflon, thermoplastic polyurethanes (TPU), polyacrylamide, styrene-butadiene-copolymers, ABS, and polymers gained from emulsion polymerization and solvent polymerization.

The process for SEC of a polymeric substance 1 according to the present invention comprises the steps a), b) and c) as mentioned above.

### Step a)

Step a) concerns the mixing of the polymeric substance 1 with a solvent, whereby a mixture M 1 is obtained.

The solvent used in step a) of the process of the present invention may be a single solvent or a mixture of two or more solvents. The selection of the solvent depends on several factors. The solvent should be able to dissolve the polymeric substance 1 as good as possible at a given temperature and concentration used in the size exclusion chromatography in step c) of the present invention. Further, the solvent should not induce any other interactions between the sample (i.e. the polymeric substance 1) and the stationary phase of the SEC column.

The principles for the selection of a suitable solvent or solvent mixture for SEC of particular polymeric substances are known by a person skilled in the art.

Suitable SEC solvents are organic solvents, water, inorganic solvents or mixtures thereof.

In the meaning of the present invention, organic solvents are carbon-based solvents (that is, they contain carbon in their molecular structure). Organic solvents can be polar and non-polar. Inorganic solvents are solvents other than water that do not have organic components in them. Inorganic solvents are usually polar solvents. Examples of inorganic solvents are ammonia, sulfuric acid, and fluoride sulfuryl chloride.

Typical SEC solvents are: water, dimethyl sulfoxide, acetonitrile, methanol, dimethylacetamide, dimethylformamide, N-methylpyrrolidone, tetrahydrofuran (THF), hexafluoroisopropanol, toluene, pentane, hexane, heptane, chloroform, dichloromethane, trichlorobenzene, dichlorobenzene, dichloroethane, acetic acid, dihydrolevoglucosenon (e.g. Cyrene^{™}), and mixtures thereof.

Mixtures of the solvents mentioned above are for example water or water buffers mixed with an alcohol, for example methanol, whereby for example up to 50 % of the alcohol may be used, or organic solvents/water mixtures.

The solvents may be modificated by the addition of e.g. salts, acids and/or bases, e.g. water-based buffers.

Less typical solvents: acetol (hydroxyaceton), ethanol, acetate, benzene, butanol, butylacetate, carbon tetrachloride, diethyl ether, diisopropyl ether, isooctane, dioxane, cyclohexane, propanol, isopropanol, xylene, MEK (methyl ethyl ketone), ethyl lactate, 2-methyl-tetrahydrofuran, MTBE (2-methoxy-2-methylpropane), cyclopentyl methyl ether, trichloroethylene, supercritical fluids, ionic fluids and green solvents, and mixtures thereof.

The solvents may be modificated by the addition of e.g. salts, acids and/or bases.

In the meaning of the present invention, green solvents are environmentally friendly solvents, or biosolvents, which are for example derived from the processing of agricultural crops. Green solvents were developed as a more environmentally friendly alternative to petrochemical solvents. Ethyl lactate, for example, is a green solvent derived from processing corn.

Preferred solvents for step a) of the process of the present invention are solvents described above as typical SEC solvents and mixtures thereof, which may be modified as mentioned above.

In the following, examples for polymer/solvent combinations are mentioned, only for illustrating:
Polymers like polyethylene glycols (PEG)/oxide (PEO) or polysaccharides (SAC) may for example be analyzed with water or water buffer, optionally with an alcohol (for example up to 50 % by weight of methanol) as a solvent.
Polymers like polystyrene (PS) or polymethyl methacrylate (PMMA) may for example by analyzed in SEC with THF, chloroform or toluene as a solvent.

As an alternative, polyethylene glycol/oxide or polymethyl methacrylate may be analyzed by SEC in mixtures of organic solvents with water or in polar organic solvents like DMF or NMP.

The solvent used in step a) for preparing the sample is generally the same as the solvent used in step c) and also the same solvent as used in step b).

The term "fully soluble" is known in the art. In the meaning of the present invention, the term "fully soluble" preferably means that no insoluble residue of the polymeric substance is left when the solvent according to step a) is used, at a given temperature. The solubility of the polymeric substance is determined as follows:
If a given amount of the polymeric sample is mixed at the temperature given in step c) with a given amount of solvent in a closed vial and shaken for 24h at the temperature given in step c) and after filtration through a 0.45µm) filter at said temperature the amount of polymer in the solvent does not change, then it is "fully soluble". If the amount of polymer in the solvent changes, then it is "not fully soluble". The amount of polymer can be determined for example spectroscopically or by gravimetry.

. The term "not fully soluble" means that an insoluble residue of the polymeric substance is left in the solvent used under the conditions mentioned above. Generally, said insoluble residue is at least 1 wt.% of the total weight of the polymeric substance 1, preferably 5 to 99 wt.%.

The concentration of the soluble part of polymeric substance 1 in the mixture M1 is generally dependent on the molecular weight and the viscosity of the polymeric substance 1 under investigation.

The principles for the selection of a suitable concentration for SEC of particular polymeric substances are known by a person skilled in the art.

The concentration of the polymeric substance 1 (soluble part) in the solvent is generally 0.01 g/l to 100 g/l, preferably 0.02 g/l to 20 g/l, more preferably 0.1 to 5 g/l.

The temperature in step a) is generally from -80 °C to 250 °C, preferably from -20 °C to 170 °C, more preferably from 15 °C to 80 °C.

The process of the present invention is carried out with polymeric substances 1, which are not fully soluble in the solvent at a given temperature, at which the SEC is performed, i.e. at the temperature of step c) of the process of the present invention. This does not necessarily mean that the mixing in step a) has to be performed at the same temperature as step c).

However, in a preferred embodiment, step a) of the process of the present invention is carried out at a temperature lower or at most 15 °C higher, preferably lower or at most 10 °C higher, more preferably lower or at most 5 °C higher ,most preferably lower or at the same temperature than step c). More preferably, step a), b) are carried out at a lower temperature or at most 15 °C higher, preferably at a lower temperature or at most 10 °C higher, more preferably at a lower temperature or at most 5 °C higher, most preferably at a lower temperature or at the same temperature as step c).

At a temperature lower than step c) preferably means at a temperature which is at most 100 °C lower, more preferably at most 80 °C lower, most preferably at most 60 °C lower than the temperature in step c.

The mixing time is generally from 2 minutes to 72 hours, preferably from 5 minutes to 48 hours, more preferably from 1 hour to 24 hours.

The mixing in step a) can be carried out in any device known by a person skilled in the art.

### Step b)

At least a part of the mixture M1 obtained in step a) (i.e. the polymeric substance 1 in a solvent, wherein the polymeric substance 1 is not fully soluble at a given temperature and concentration of step c)) is subjected to an ultrasonic treatment in step b), whereby a mixture M2A comprising a polymeric substance 2 is obtained.

The mixture which is ultrasonificated in step b) of the present invention is named mixture M1A.

It is possible to subject the whole mixture M1 obtained in step a) to the ultrasonic treatment in step b).

Alternatively, a part of the mixture M1 obtained in step a) is ultrasonificated in step b). In this case, the ultrasonificated substance in mixture M2A (polymeric substance 2) is used as a reference substance and compared with the polymeric substance 1 of the present invention which is not ultrasonificated prior to subjection of the mixture to size exclusion chromatography. Further explanation is given below.

Generally, the amount of the part of mixture M1 which is subjected to ultrasonification in step b) (i.e. mixture M1A) is not of particular relevance. Usually, at least 0.05 mL of the mixture M1 obtained in step a) are ultrasonificated in step b), preferably 0.1 to 100 mL, more preferably 0.5 mL to 20 mL.

The ultrasonic treatment (ultrasonification) is generally carried out at a temperature of 0 °C to 100 °C, preferably 5 °C to 50 °C, more preferably 10 °C to 40 °C. Preferably, the ultrasonic treatment in step b) of the process of the present invention is carried out at a lower temperature or at most 15 °C higher, preferably at a lower temperature or at most 10 °C higher, more preferably at a lower temperature or at most 5 °C higher, most preferably at a lower temperature or at the same temperature as step c).

The ultrasonic treatment in step b) is generally carried at an irradiation time of at least 5 minutes, preferably from 10 minutes to 48 hours, more preferably from 15 minutes to 24 hours, most preferably from 30 minutes to 24 hours.

The ultrasonic frequency in the ultrasonic treatment in step b) is generally from 10 to 500 kHz, preferably 20 to 200 kHz, more preferably 20 to 50 kHz.

The output in the ultrasonic treatment in step b) is generally at least 10 W, preferably 20 W to 1000 W, more preferably 100 W to 1000 W.

The ultrasonic treatment in step b) can be usually performed in any ultrasonic generator known in the art.

After the ultrasonic treatment in step b) of the process of the present invention, a mixture M2A comprising a polymeric substance 2 is obtained. The chemical composition and the detector response and dn/dc of the polymeric substance 2 are identical with those of the polymeric substance 1 (i.e. the sample). The polymeric substance 2 is therefore suitable as a reference substance for the polymeric substance 1 (the sample itself).

Preferably, the polymeric substance 2 in the mixture M2A obtained after ultrasonic treatment in step b) of the present invention is soluble in the solvent mentioned in step a) at a given temperature in step c) to a higher weight fraction than the polymeric substance 1, more preferably the polymeric substance 2 is soluble in the solvent to a weight fraction of at least 90 wt.%, preferably 95 to 99 wt.%, more preferably fully soluble, in the solvent mentioned in step a) at a given temperature in step c) of the present invention.

In a preferred embodiment of the process of the present invention, the mixture M1 obtained in step a) is split into two parts, M1A and M1B, wherein
i) the mixture M1A is subjected to the ultrasonic treatment according to step b) described above, whereby the mixture M2A is obtained, which mixture M2A is subjected to the size exclusion chromatography according to step c); and
ii) the mixture M1B is filtered, whereby a filtrate M2B is obtained, and the filtrate M2B is subjected to the size exclusion chromatography according to step c).

Preferably, step ii) is carried out at a lower temperature or at most 15 °C higher, preferably at a lower temperature or at most 10 °C higher, more preferably at a lower temperature or at most 5 °C higher, most preferably at a lower temperature or at the same temperature as step c).

The filtration in step ii) can be carried out as known by a person skilled in the art. Also, suitable filters are known by a person skilled in the art.

The filtrate M2B comprises the soluble part of the polymeric substance 1 in the solvent mentioned in step a) of the present invention.

Preferably, the SEC analysis of the mixture M2A and of the filtrate M2B (i.e. step c)) are carried out identically (i.e. at identical conditions) for comparative reasons.

By splitting the mixture M1 and treating the two parts M1A and M1B as mentioned above, and subsequent SEC analysis according to step c) two chromatograms are obtained. By comparison of the SEC of the polymeric substance 2 (reference substance) present in the mixture M2A with the SEC of the soluble fraction of polymeric substance 1 (sample), present in the filtrate M2B, from the ratio of the two chromatograms, the low molecular weight proportions can be calculated exactly and a better (more exact) determination of the molecular weight mean value of the sample (polymeric substance 1) is possible.

However, even from the ultrasonically treated reference substance alone (polymeric substance 2), a lower limit for the molecular weight mean values and an upper limit for the low molecular weight fraction is directly accessible (without comparison with the measurement of the soluble fraction of the sample (polymeric substance 1)). This simplified analysis is in many cases sufficient to meet the regulatory requirements of specifying the low molecular weight fractions if these limits are met.

### Step c)

Step c) concerns subjecting the mixture M2A obtained in step b) to size exclusion chromatography at a given temperature and at a given concentration.

The given temperature as well as the given concentration vary depending on the polymeric substance 1 employed and on the solvent employed in the process of the present invention.

The temperature in step c) ("given temperature") is generally -80 °C to 250 °C, preferably -20 °C to 170 °C, more preferably 15 °C to 80 °C. Preferably, step c) is carried out at a higher temperature or at the same temperature as step a) and step b).

At a temperature higher than step a) and step b) preferably means at a temperature which is at most 100 °C higher, more preferably at most 80 °C higher, most preferably at most 60 °C higher than the temperature in step a) and step b).

The concentration of the polymeric substance 2 in the mixture M2A as well as the concentration of the polymeric substance 1 in mixture M2B (filtrate) are generally dependent on the molecular weight and the viscosity of the polymeric substance 1 under investigation, whereby the concentration of the polymeric substance 1 in mixture M2B (filtrate) is the same as the concentration of the soluble part of polymeric substance 1 in mixture M1 in step a). The concentration of the polymeric substance 2 in mixture M2A is higher than the concentration of the soluble part of the polymeric substance 1 in mixture M1, since at least a part of the insoluble part of polymeric substance 1, preferably a weight fraction of at least 50 wt.%, more preferably 90 to 99 wt.%, most preferably, the complete insoluble part of the polymeric substance 1 is dissolved in mixture M2A.

The concentration of polymeric substance 2 ("given concentration") in the solvent in the mixture M2A is generally 0.02 g/l to 100 g/l, preferably 0.03 g/l to 20 g/l, more preferably 0.1 g/l to 5 g/l.

The concentration of the polymeric substance 1 after filtration in the solvent in the mixture M2B is the same as the concentration of the soluble part of polymeric substance 1 in step a).

Size exclusion chromatography is known by a person skilled in the art. Suitable columns are commercially available, and it is also known by a person skilled in the art what kind of column is useful for what kind of polymer.

The separation of the polymers in mixture M2A and M2B or generally of the sample takes place inside the column, a hollow tube tightly packed with extremely small porous beads, designed to have pores of well-defined size, typically made from polymers that have been cross-linked to make them insoluble or inorganic materials, such as spherical silicates.

The columns vary in length, for example from 50 mm to 600 mm, and internal diameters, for example of 2 mm to 25 mm, depending on their intended use.

The type of bead in the column is controlled to match different applications. The particle diameters of the beads are for example between 1 to 20 µm. The pore sizes range for example from 50 to 1000000 Å.

Columns are generally packed with different sized particles with different sized pores primarily for different molecular weight ranges. The reason for this specificity is that the nature of the sample and solvent determines which column configuration will provide the best analytical result, which is why there are many different combinations of particle size and pore size. It is generally possible to use one column for SEC but it is also possible to employ columns in combinations of two or more, especially two or three columns to improve the resolution of the system.

In case that the mixture M1 obtained in step a) is split into two parts, M1A and M1B, wherein one part (M1A) is subjected to ultrasonic treatment and the other part (M1B) is filtered, the mixture M2A obtained after ultrasonic treatment and the filtrate M2B are generally subjected - subsequently - to the same SEC column, at the same conditions for comparative reasons.

The present invention further relates to a reference substance, preferably for size exclusion chromatography, of a polymeric substance 1, obtainable by
a) mixing the polymeric substance 1 with a solvent, whereby a mixture M1 is obtained; and
b) ultrasonic treatment of the mixture M1 obtained in step a), whereby the reference substance for the polymeric substance 1 is obtained.

Preferably, the polymeric substance 1 is not fully soluble in the solvent mentioned in step a) at a given temperature of subjecting the polymeric substance 1 to size exclusion chromatography or to another analysis method. However, the inventive reference substance is preferably fully soluble in the solvent mentioned in step a) at the temperature of subjecting the reference substance to size exclusion chromatography or to another analysis method.

Suitable temperatures, concentrations, solvents, polymeric substances 1 and further reaction parameters in steps a) and b) are the same as mentioned for steps a) and b) in the process described above.

The inventive reference substance based on an ultrasonic treatment of the polymeric substance 1, which is not fully soluble in the solvent of the mixing step a) at a given temperature and a given concentration in the step of subjecting said substance to size exclusion chromatography, corresponds to polymeric substance 2 of step b) of the inventive process.

The present invention further relates to the use of polymeric substance 2 obtained in step b) of the process according to the present invention as a reference substance for polymeric substance 1, preferably in size exclusion chromatography.

A further embodiment of the present invention relates to an SEC column comprising the reference substance according to the present invention. Suitable SEC columns are known by a person skilled in the art.

The present invention enables SEC measurements of polymeric substances which are not fully soluble in the solvent used in SEC at a given temperature of the SEC. The analysis report obtained after said process can for example be used to register polymeric products (polymeric substance 1) for certain markets like China, Korea, Europe, US, Canada, etc. This registration is required to sell the polymeric substances to those markets.

The inventive reference substance according to the present invention is not only useful in SEC but also in other techniques (analysis methods) like:
- determination of dn/dc for polymers (different apparatus and methods possible)
- chromatography of polymers (other than SEC, e.g. LAC, GEPC and the like)
- field-flow-fractionation
- light scattering of polymers (in batch or coupled to fractionation techniques)
- ultracentrifugation of polymers
- viscosity measurements of polymers (in batch, solution or coupled to fractionation techniques)
- polymer analytics and solution in general

Next, the invention is explained in more detail in accordance with the following example, which should not be construed as limiting the scope of the invention.

### Example

A dispersion of a partially crosslinked styrene-butadiene-copolymer, was dissolved in THF at room temperature, whereby the copolymer was not fully soluble.

The mixture obtained was split into two parts.

One part (first part of the sample) was filtered and analyzed directly by SEC.

The second part was treated with ultrasound for 5 hours in THF at room temperature and subsequently analyzed by SEC in the same way as the first part.

The SEC measurements were performed in THF on SEC columns filled with styrene-divinylbenzene separation material at room temperature.

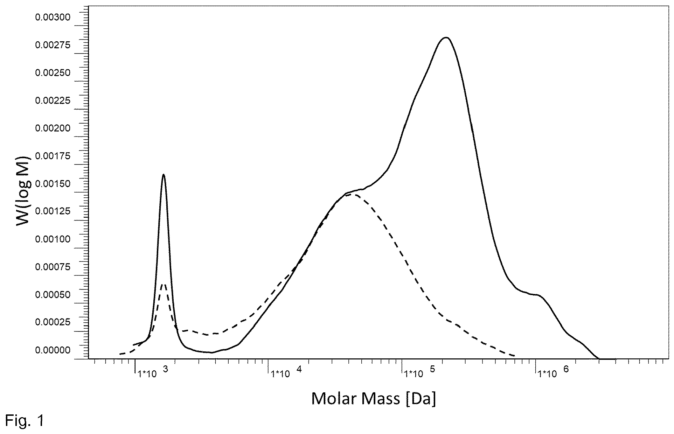

In Figure 1 it is shown that without ultrasonic treatment only a part of the sample which is not fully soluble in the solvent for SEC at a given temperature of SEC is analyzed (in this case roughly 50 %). With ultrasonic treatment, the peak area increases. Thus, the whole portion of the sample can be analyzed with SEC.

The ultrasonic treatment destroys the molecular weight distribution curve but the peak area is reliable. Therefore, the curve from the original measurements (dotted line, first part of the sample) should be used and set into relation with the peak area from the treated curve (solid line, second part of the sample) to determine the oligomeric amounts.

## Claims

1. A process for size exclusion chromatography of a polymeric substance 1 comprising the following steps
a) Mixing the polymeric substance 1 with a solvent, whereby a mixture M1 is obtained;
b) Ultrasonic treatment of at least a part of the mixture M1 obtained in step a), which is named mixture M1A, whereby a mixture M2A comprising a polymeric substance 2 is obtained; and
c) Subjecting the mixture M2A obtained in step b) to size exclusion chromatography, at a given temperature and at a given concentration,
wherein the polymeric substance 1 is not fully soluble in the solvent at the temperature and concentration in step c).

2. The process according to claim 1, wherein at least 2 wt.%, preferably at least 5 wt.%, more preferably 5 wt.% to 95 wt.% of the polymeric substance 1 have a molecular weight of at least 5 000 000 Da, determined according to OECD118 .

3. The process according to claim 1 or 2, wherein the solvent is an organic solvent, water, an inorganic solvent or a mixture thereof.

4. The process according to any one of claims 1 to 3, wherein the polymeric substance 1 is present in the solvent in a concentration of 0.01 g/l to 100 g/l, preferably 0.02 g/l to 20 g/l.

5. The process according to any one of claims 1 to 4, wherein the temperature in step a) is -80 °C to 250 °C, preferably from -20 °C to 170 °C.

6. The process according to any one of claims 1 to 5, wherein at least 1 wt.%, preferably 5 to 99 wt.% of the polymeric substance 1 are not soluble in the solvent.

7. The process according to any one of claims 1 to 6, wherein step a), preferably step a) and step b) are carried out at a temperature lower or at most 15 °C higher, preferably lower or at most 10 °C higher, more preferably lower or at most 5 °C higher, most preferably lower or at the same temperature as step c).

8. The process according to any one of claims 1 to 7, wherein the ultrasonic treatment is carried out at an irradiation time of at least 5 minutes, an ultrasonic frequency of 10 to 500 kHz, and an output of at least 10 W.

9. The process according to any one of claims 1 to 8, wherein the polymeric substance 2 of the mixture M2A in step b) is soluble to a higher weight fraction than the polymeric substance 1 in the mixture M1, most preferably the polymeric substance 2 of the mixture M2A in step b) is fully soluble, in the solvent mentioned in step a) at a given temperature in step c).

10. The process according to any one of claims 1 to 9, wherein the polymeric substance 2 obtained step b) is used as reference substance for the polymeric substance 1.

11. The process according to any one of claims 1 to 10, wherein the mixture M1 obtained in step a) is split into two parts, M1A and M1B, wherein
i) the mixture M1A is subjected to the ultrasonic treatment according to step b), whereby the mixture M2A is obtained, and the mixture M2A is subjected to the size exclusion chromatography according to step c); and
ii) the mixture M1B is filtered, whereby a filtrate M2B is obtained, and the filtrate M2B is subjected to the size exclusion chromatography according to step c).

12. The process according to claim 11, wherein the filtration of mixture M1B and preferably also the mixing in step a) are carried out at a temperature lower or at most 15 °C higher, preferably at a temperature lower or at most 10 °C higher, more preferably at a temperature lower or at most 5 °C higher, most preferably at maximum the same temperature as the size exclusion temperature in step c).

13. A reference substance, preferably for size exclusion chromatography, of a polymeric substance 1, obtainable by
c) mixing the polymeric substance 1 with a solvent, whereby a mixture M1 is obtained; and
d) ultrasonic treatment of the mixture M1 obtained in step a), whereby the reference standard for the polymeric substance 1 is obtained.

14. A size exclusion chromatography column comprising the reference standard according to claim 13.

15. Use of a polymeric substance 2 obtained in step b) of the process according to any one of claims 1 to 10 as a reference substance for polymeric substance 1, preferably in size exclusion chromatography.
